(19) Europäisches Patentamt
European Patent Office
Office européen des brevets

(11) Veröffentlichungsnummer: **0 210 391**
**A1**

(12) **EUROPÄISCHE PATENTANMELDUNG**

(21) Anmeldenummer: 86107897.0

(22) Anmeldetag: 10.06.86

(51) Int. Cl.[4]: C 07 C 175/00
C 11 B 9/00, A 61 K 7/46

(30) Priorität: 24.06.85 CH 2666/85

(43) Veröffentlichungstag der Anmeldung:
04.02.87 Patentblatt 87/6

(84) Benannte Vertragsstaaten:
CH DE FR GB LI NL

(71) Anmelder: L. GIVAUDAN & CIE Société Anonyme

CH-1214 Vernier-Genève(CH)

(72) Erfinder: Helmlinger, Daniel, Dr.
Ticheirütistrasse 18
CH-8044 Gockhausen(CH)

(74) Vertreter: Urech, Peter, Dr. et al,
Grenzacherstrasse 124 Postfach 3255
CH-4002 Basel(CH)

(54) **Verfahren zur Herstellung von cyclischen Verbindungen.**

(57) Es wird ein Verfahren zur Herstellung von mehrheitlich in der trans-Form vorliegenden Verbindungen der Formel

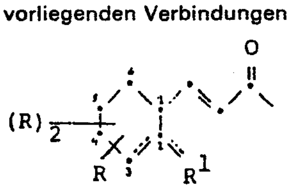

I

worin das Symbol R für eine Methylgruppe steht, $R^1$ für Methyl oder Methylen steht, $(R)_2$ zwei Methylgruppen in geminaler Stellung in 3,4,5 oder 6-Stellung des Moleküls darstellt und eine der gestrichelt gezeichneten Linien eine zusätzliche Bindung ist, beschrieben.

EP 0 210 391 A1

Ref. 6510/224

L. GIVAUDAN & CIE Société Anonyme, Vernier-Genève (Schweiz)

## Verfahren zur Herstellung von cyclischen Verbindungen

Die Erfindung betrifft ein Verfahren zur Herstellung von mehrheitlich neuen, ungesättigten, cyclischen Verbindungen durch eine en-Reaktion [bzgl. en-Reaktion siehe z.B. Römpps Chemielexikon, Franckh'sche Verlagshandlung Stuttgart, 1981, 8. Auflage, Band 2, Seite 1139]. Diese Verbindungen sind die mehrheitlich in der trans-Form vorliegenden Verbindungen der Formel

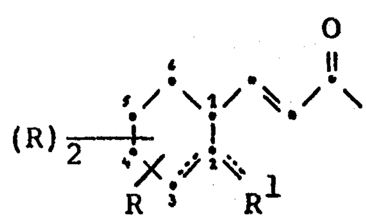

I

Ur/30.04.86

worin das Symbol R für eine Methylgruppe steht, $R^1$ für Methyl oder Methylen steht, und $(R)_2$ zwei Methylgruppen in geminaler Stellung in 3, 4, 5 oder 6-Stellung des Moleküls darstellt und eine der gestrichelt gezeichneten Linien eine zusätzliche Bindung ist.

Die zusätzliche Bindung ist also im Falle $R^1$= Methylen, eine der beiden Methylenbindungen bzw., im Falle $R^1$= Methyl, eine weitere Bindung zwischen den Kohlenstoffatomen 2 und 3 im Ring.

Die Verbindungen der Formel I stellen Riechstoffe dar.

Das Verfahren ist dadurch gekennzeichnet, dass man eine Verbindung der Formel

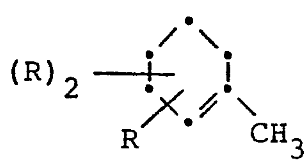

II

worin R und $(R)_2$ obige Bedeutungen besitzen, in Gegenwart einer schwachen Lewis-Säure mit 3-Butin-2-on behandelt.

Die Erfindung betrifft auch die neuen Riechstoffe der Formel I. Es handelt sich dabei um die Verbindungen der Formel I mit der Ausnahme von mehrheitlich in der trans-Form vorliegendem α-Iron und γ-Iron.

In den Verbindungen der Formel I sind asymmetrische Kohlenstoffatome vorhanden, so dass die Verbindungen als optische Antipoden vorliegen können. Die Formel I soll

demnach alle möglichen isomeren Formen umfassen, wobei aber definitionsgemäss in jedem Fall der Anteil an trans-Isomeren (angesprochen wird diesbezüglich die relative Stereochemie der Substituenten am Ring) immer überwiegt, d.h. mehr als 50%, insbesondere sogar mehr als ca. 95% beträgt.

Unter "schwacher Lewissäure" soll eine Lewissäure verstanden werden, deren Säurestärke grössenordnungsmässig derjenigen der Salze der Elemente der Gruppe 2 b des Periodischen Systems entspricht; siehe auch D.P.N. Satchell, Q. Rev. Chem. Soc. 25 (1971), Seiten 171 - 199.

Beispiele von solchen schwachen Lewissäuren sind:

Zink-Salze, wie Zink-Halogenide, z.B. das Chlorid, Jodid, Bromid.

Die Umsetzung der Verbindung der Formel II mit dem 3-Butin-2-on erfolgt zweckmässigerweise in einem inerten Verdünnungsmittel, insbesondere einem organischen, aprotischen Lösungsmittel, wie einem aliphatischen oder aromatischen, halogenierten Kohlenwasserstoff,z.B. Methylenchlorid, Chloroform oder Chlorbenzol.

Das Verhältnis Verbindung II/3-Butin-2-on beträgt zweckmässigerweise ca. 1:1, doch ist auch ein Ueberschuss an Verbindung II oder an 3-Butin-2-on möglich.

Diese Umsetzung wird im allgemeinen unter milden Bedingungen, nämlich bei Temperaturen zwischen 0°C und 30°C, vorzugsweise bei Raumtemperatur, durchgeführt, doch sind auch noch höhere oder noch tiefere Temperaturen möglich.

Die Menge der Lewissäure ist nicht kritisch, sie beträgt im allgemeinen ca. 10 bis ca. 200 Molprozent, bezogen auf die Menge Verbindung der Formel II.

Die Reaktionsdauer beträgt zweckmässigerweise 1 bis 12 Tage.

Der vollständige Ablauf der Reaktion kann beispielsweise gaschromatographisch oder dünnschichtchromatographisch kontrolliert werden.

Die Isolierung und die Reinigung der so hergestellten Verbindungen der Formel I, bzw. Gemische solcher Verbindungen, kann nach an sich bekannten Methoden erfolgen, wie z.B. Chromatographie, z.B. Säulenchromatographie, Destillation, Kristallisation.

Das erfindungsgemässe Verfahren, das mit grosser Stereoselektivität abläuft - im Reaktionsgemisch findet sich immer ein überwiegender Anteil des trans-Isomeren - gestattet insbesondere auch die Herstellung der entsprechenden Irone, also des trans-α-Irons I' und des trans--γ-Irons I".

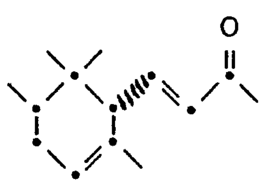

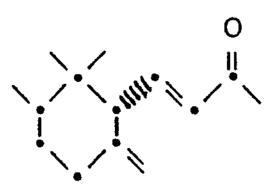

I'

I"

Die Ketone I stellen mehrheitlich farblose bis leicht gelblich gefärbte Flüssigkeiten dar, sind unlöslich in Wasser, aber löslich in organischen Lösungsmitteln, wie z.B. Alkoholen, Aethern, Ketonen, Estern, Kohlenwasserstoffen und halogenierten Kohlenwasserstoffen.

Die Ausgangsmaterialien der Formel II sind bekannt oder können nach an sich bekannten Methoden hergestellt werden.

Die Verbindungen der allgemeinen Formel I weisen, wie oben gesagt, besondere organoleptische Eigenschaften auf.

Die Verbindungen der Formel I zeichnen sich insbesondere durch ironartige, tabakartige, fruchtige und holzige Geruchsnoten aus. Im Vordergrund steht insbesondere eine Kombination von holzigen und oder irisartigen, iononartigen Noten und ausgesprochener Haftfestigkeit.

Die Verbindungen der Formel I eignen sich auf Grund ihrer Geruchsnoten insbesondere beim Verstärken von irisartigen Noten und als Verstärker von Tabaknoten. Daneben eignen sie sich zur Modifizierung einer ausgesprochen breiten Palette von bekannten, z.B.

a) blumigen Kompositionen, in denen z.B. die warmen Irisnoten verstärkt zum Ausdruck kommen sollen (z.B. für Herren-Colognes),

b) des weiteren aber auch von Chypre-Kompositionen (Extrait-Typen, Kompositionen der femininen Richtung), von

c) Tabak- und Holz- und Fougère-Kompositionen, (Extrait--Typen der masculinen Richtung) und von

d) Kompositionen mit grünen Noten, wo insbesondere eine erwünschte Verstärkung und Abrundung und harmonisierende Effekte erzielt werden.

Als Riechstoffe eignen sich die Verbindungen I auf Grund ihrer oben beschriebenen, originellen Noten, insbe-

sondere in Kombination mit einer Reihe von natürlichen und synthetischen Riechstoffen, wie z.B.

- Naturprodukte, wie Angelikawurzöl, Bergamotteöl, Calmusöl, Cassis absolue, Castoreum, Cedernöl, Ciste labdanum, Corianderöl, Eichenmoos, Galbanumöl, Geraniumöl, Grapefruit-Extrakten, Fichtenöl, Jasmin Absolue, Lavendelöl, Mandarinenöl, Muskatellersalbei, Narzissen absolue, Orangenöl, Patchouliöl, Rosenöl, Sandelholzöl, Verveine absolue, Vetiveröl, Ylang-Ylang-Oel, Zitronenöl, usw.

- Aldehyde, wie Benzaldehyd, $C_{10}$-, $C_{11}$-, $C_{14}$-Aldehyd, Citral, Citronellal, Cyclal C (1,3-Dimethyl-cyclohex-1-en 4(und 5)-carboxaldehyd), Cyclamenaldehyd, 2,4-Decadienal, 2,6-Dimethyl-5-hepten-1-al, Hydroxycitronellal, Isovaleraldehyd, Lyral, Methylnonylacetaldehyd, n-Nonanal, n-Octanal, p-tert. Butyl-α-methyl-hydro--zimt-aldehyd, trans-2-cis-6-Nonadienal, trans-2-Hexenal, trans-2-Octenal, usw.

- Ketone, wie Acetanisol, Allyljonon, α-Jonon, ß-Jonon, Campher, Carvon, Menthon, p-Methylacetophenon, Methylamylketon, Methyljonon, 4-(para-Hydroxyphenyl)-2-butanon, Pulegon, etc.

- Acetalen und Ketalen, wie Phenylacetaldehyd-dimethylacetal, Phenylacetaldehyd-glycerinacetal, 2-Methyl-1,3--dioxolan-2-äthylacetat, Capronaldehyd-dimethylacetal, Acetal R (gemischtes Acetal von Acetaldehyd mit Phenyläthylalkohol und n-Propanol), usw.

- Aethern, wie Eugenolmethyläther, Methyl-1-methyl-cyclododecyläther, Anethol, Estragol, Rosantolène (Methyläthylsaligenin), usw.

- Phenolkörpern, wie Vanillin, Eugenol, Creosol, Chavicol, usw.

- Alkoholen, wie Butanol, Benzylalkohol, cis-3-Hexenol, cis-6-Nonenol, Citronellol, Farnesol, Geraniol, Linalool, n-Hexanol, Nerol, Nerolidol, Patchone (4-tert.-Butylcyclohexanol), Phenyläthylalkohol, Rhodinol, Terpineol, trans--2-cis-6-Nonadienol, Zimtalkohol, usw.

- Ester, wie Aethylacetat, Aethyl-α-methyl-phenylglycidat, Aethylformiat, Aethylpalmitat, Amylsalicylat, Benzylacetat, Benzylsalicylat, Cinnamylformiat, Dimethylbenzylcarbinylacetat und butyrat, Geranylacetat, Hexylsalicylat, Isoamylacetat, Isobutylacetat, Linalylacetat, Linalylanthranilat, Maltylisobutyrat, Methyldihydrojasmonat, Myraldylacetat [R] (Givaudan), n-Amylbutyrat, n-Amylvalerianat, Styrallylacetat, t-Butylcyclohexylacetat, Terpenylacetat, usw.

- Lactone, wie Cumarin, γ-Decalacton, γ-Undecalacton, γ-Nonalacton, ϕ-Decalacton, ϕ-Octalacton, usw.

-Säuren, wie Geranylsäure, Citronellylsäure, Zimtsäure, Phenylessigsäure, usw.

- schwefelhaltigen Verbindungen, wie p-Menthan-8-thiol-3--on, Dimethylsulfid und anderen Sulfiden und Disulfiden, usw.

- stickstoffhaltigen Verbindungen, wie Methylanthranilat, Indol, Isobutylchinolin, verschiedenen Pyrazinen, 5-Methyl-heptan-3-on-oxim, Nitromoschus, usw.

- <u>verschiedene weitere, in der Parfümerie oft benützten Komponenten</u>, wie Galaxolid, Keton-Moschus, macrocyclische Moschuskörper wie Musk 174 [R] (12-Oxahexadecanolid), polycyclische Moschuskörper wie Fixolid, Sandela (Isocamphylcyclohexanol).

Die Verbindungen der Formel I lassen sich in weiten Grenzen einsetzen, die beispielsweise von 0,1 (Detergentien) - 50% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 20%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Desodorantien, Detergentien, Raumparfums, etc.).

Die Verbindungen I können demgemäss bei der Herstellung von Kompositionen und - wie obige Zusammenstellung zeigt - unter Verwendung einer breiten Palette bekannter Riechstoffe, verwendet werden. Bei der Herstellung solcher Kompositionen können die oben aufgeführten bekannten Riechstoffe nach dem Parfümeur bekannter Art und Weise verwendet werden, wie z.B. aus W.A. Poucher, Perfumes, Cosmetics and Soaps <u>2</u>, 7. Auflage, Chapman und Hall, London, 1974 hervorgehend.

Es sind zwar den neuen Verbindungen der Formel I strukturell ähnliche Verbindungen bekannt.

So beschreiben z.B. Stoll et al. in Helv. Chim. Acta 30, Fasc. 7 (1947), Seiten 2213 - 15 das 4-Methyl-ionon der Formel

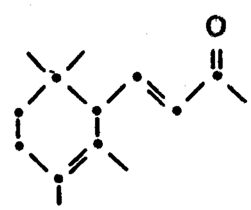

III

und seinen Geruch, und auf Seite 2216 - 20 das 5-Methyl-ionon der Formel

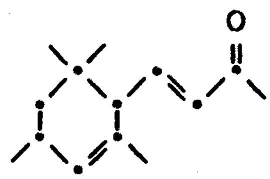

IV

und seinen Geruch.

Erstere Verbindung stellt aber das Produkt einer 8-stufigen Synthese und die zweitgenannte Verbindung das Produkt einer 9-stufigen Synthese dar. Die völlig unbefriedigende Synthese der zweitgenannten Verbindung [siehe diesbezüglich auch DT-OS 3,327,304, Seite 3] wurde zwar neulich scheinbar verbessert, siehe diese DT-OS 3,327,304 vom 7. Februar 1985; der Nachteil der neuen Synthese ist aber nach wie vor der, dass dabei von den schwer zugänglichen Methylhalogenpentenen ausgegangen wird, nämlich Verbindungen, die nur schwierig und mit äusserst unbefriedigenden Ausbeuten anfallen (E.N. Marvell, J.W. Nelson, J. Org. Chemistry, 45, (1980), 5217 - 5218).

Was die erfindungsgemässe Herstellung der in der trans-Form vorliegenden α- und γ-Irone betrifft, waren auch diese Verbindungen bisher nur mittels mehrstufigen und umständlichen Synthesen zugänglich, siehe z.B.

J. Garnero, D. Joulain, Bull. Soc. Chim. France, II, 15/16 (1979): 6-stufige Synthese für trans-α-Iron,

O. Takazawa, K. Kogami, K. Hayashi, Bull. Chem. Soc. Japan, <u>58</u>, 389-390 (1985): 7-stufige Synthese für trans--α-Iron,

Leyendecker, Comte, Dissertation Strasbourg (1984): 5-stufige Synthese für ein Gemisch von cis- und trans-γ--Iron,

M. Miyashita, N. Makino, M. Singh, A. Yoshikoshi, J. Chem. Soc. Perkin Trans. I, 1303 - 1309 (1982): 20-stufige Synthese für optisch aktives trans-α-Iron,

T. Kitahara, K. Tanida, K. Mori, Agric. Biol. Chem. <u>47</u> (3), 581-586 (1983): ebenfalls vielstufige Synthese für trans-α-Iron.

Die verhältnismässig kurze Synthese von V. Rautenstrauch, B. Willhalm, W. Thommen und G. Ohloff, Helv. Chim. Acta, 67 (1), Nr. 38, 325 - 331 (1984), andererseits, liefert das trans-α-Iron nur im Gemisch mit cis-α-Iron.

## <u>Beispiel 1</u>

Eine Lösung von 10 g 1,3,3,4-Tetramethyl-cyclohex-1-en und 6,1 g 3-Butin-2-on in 80 ml Methylenchlorid wird in Gegenwart von 6,1 g Zinkchlorid 11 Tage gerührt. Dann wird auf Eis gegossen und mit Aether extrahiert, über Magnesiumsulfat getrocknet und eingedampft. Das Rohprodukt (13,4 g) wird über 400 g Kieselgel 60 Merck (Korngrösse 0,04 - 0,063 mm) chromatographiert; die Elution erfolgt mit 5% und 10% Aether in Hexan. Man erhält so zunächst 1 g 1,3,3,4-Trimethyl-cyclohex-1-en und hierauf 9 g eines Pro-

duktes, das gemäss gaschromatographischer Analyse 42% trans-α-Iron, 21% trans-γ-Iron, 15% trans-trans-4--(2',4',4',5'-Tetramethyl-cyclohex-2'-en-1'-yl)-but-3-en--2-on und 15% trans-trans-4-(4',4',5'-Trimethyl-2'-methylen-cyclohex-1'-yl)-but-3-en-2-on darstellt.

Die einzelnen Verbindungen können durch eine weitere chromatographische Trennung auf 200 g Kieselgel 60 Merck (Korngrösse 0,04 - 0,063 mm + 10% $AgNO_3$) durch Elution mit 2,7 und 10% Aether in Hexan isoliert werden.

## Beispiel 2

Eine Lösung von 2,5 g 1,3,3,4-Tetramethyl-cyclohex-1-en und 1,1 g 3-Butin-2-on in 25 ml Methylenchlorid wird in Gegenwart von 7,1 g Zinkjodid 6 Tage bei Raumtemperatur gerührt. Hierauf wird auf Wasser gegossen, mit Aether extrahiert und über Magnesiumsulfat getrocknet. Das Rohprodukt (8,45 g) enthält gemäss gaschromatographischer Analyse 72% trans-α-Iron, 19% trans-γ-Iron und 3% ß-Iron. Durch Chromatographie auf 210 g Kieselgel 60 Merck (Korngrösse 0,04 - 0,063 mm) erhält man durch Eluieren mit Hexan 0,75 g 1,3,3,4-Tetramethyl-cyclohex-1-en. Durch Eluieren mit 10% Aether in Hexan erhält man 1,15 g eines Gemisches von trans-α-Iron und trans-γ-Iron. Diese 2 Verbindungen können einzeln durch eine weitere Chromatographie über 200 g Kieselgel 60 Merck (Korngrösse 0,04 - 0,063 mm), 10% $AgNO_3$ (Elution 2,7, 10% Aether) isoliert werden.

## Beispiel 3

a) Aus 1,1 g Magnesium, 6,3 g Methyljodid und 20 ml Aether wird eine Grignardlösung hergestellt. Dazu werden 5

g 3,3,4-Trimethyl-cyclohexanon (in 15 ml Aether) zugetropft. Das Reaktionsgemisch wird während 3,5 Stunden bei Rückflusstemperatur gehalten, danach mit Eis versetzt und mit 2 N Salzsäure angesäuert. Hierauf wird mit Aether extrahiert und mit einer gesättigten Kochsalzlösung gewaschen. Nach dem Eindampfen erhält man 5 g 1,3,3,4-Tetramethyl-cyclohexan-1-ol.

b) 5 g 1,3,3,4-Tetramethyl-cyclohexan-1-ol werden zusammen mit 1,9 g Oxalsäure 1,5 Stunden bei 120°C erhitzt. Danach wird das Produkt am Wasserstrahlvakuum kugelrohrdestilliert. Man erhält auf diese Weise 3,5 g eines Gemisches von 1,3,3,4-Tetramethyl-cyclohex-1-en und 1,4,5,5-Tetramethyl-cyclohex-1-en.

c) Eine Lösung von 3,4 g eines Gemisches von 1,3,3,4-Tetramethyl-cyclohex-1-en und 1,4,5,5-Tetramethyl-cylcohex-1-en, im Verhältnis 1:3, und 1,8 g 3-Butin-2-on in 30 ml Methylenchlorid werden in Gegenwart von 5,2 g Zinkchlorid zwei Tage bei Raumtemperatur gerührt. Dann wird auf Eis gegossen, die wässrige Phase mit Aether extrahiert, die organische Phase über Magnesiumsulfat getrocknet und eingedampft. Der Rückstand (4,3 g) wird über 120 g Kieselgel 60 Merck (Korngrösse 0,04 - 0,063 mm) mit 5% Aether in Hexan chromatographiert. Man erhält 2,6 g eines Gemisches, das über 200 g Kieselgel 60 Merck (Korngrösse 0,04 - 0,063 mm)/10% $AgNO_3$ mit 7% und 10% Aether in Hexan eluiert wird. Auf diese Weise werden 95% reines trans-trans-4-(2',4',4',5'-Tetramethyl-cyclohex-2'-en-1'-yl)-but-3-en-2-on und trans-trans-4-(4',4',-5'-Trimethyl-2'-methylen-cyclohex-1'-yl)-but-3-en-2-on erhalten.

Spektrale Daten von trans-trans-4-(2',4',4',5'-Tetramethyl-cyclohex-2'-en-1'-yl)-but-3-en-2-on:

$^1$H-NMR: ($CDCl_3$ 400 MHz): 0,79 und 0,96 (2s, $(CH_3)_2$ c (4')); 0,86 (d, J=7, $CH_3$-C (5')); 1,425 (ddd, J (6'eq, 1'eq)=5, J(6'eq, 6'ax)=13, J (6'eq, 5'ax)=3, H eq - C (6)); 1,5 (m, J (5'ax, 6'ax)=12, J (5'ax, 6'eq)=3, J (5'aax $H_3C$), H - C (5')); 1,68 (ddd, J (6'a, H's)=12, J (6'ax 6'e)=13, J (6'a 1)=6, Hax - C (6)); 2,265 (s, $CH_3$ - CO); 2,72 (ddd, J (1', 4)=7, J (1', 6ax)=6, J (1', 6eq)=2,3; H - C (1')); 5,28 (s, H - C (4)); 6,01 (dd, J = 0,5, J = 16, H - C (3)); 6,74 (dd, J (4,3)=16, J (4,1')=8, H - C (4));

IR: (flüssig), 1700, 1680, 1620, 1360, 1250, 990 cm $^{-1}$

MS: $M^+$ 206 (13), 191 (51), 173 (7), 163 (16), 149 (22), 133 (38), 121 (53), 107 (30), 95 (41), 91 (27), 79 (13), 71 (24), 67 (13), 55 (33), 43 (100).

trans-trans-4-(4',4',5'-Trimethyl-2'-methylen-cyclohex-1'-yl)-but-3-en-2-on.

$^1$H-NMR ($CDCl_3$ 400 MHz): 0,727 und 0,915 (2 s, $(CH_3)_2$ C (4')); 0,85 (d, J=7, $CH_3$ - C (5')); 1,87 und 2,012 (2d, J (6,6)=13, J (6,6=13), $H_2$ - C (3')); 2,26 (s, $CH_3$ - (C2)); 3,132 (m, H - C (1)); 4,755 (m, $H_2C$ = C2)); 6,085 (dd, J (3,4)=16, J (3,1)=2, H-C (3)); 6,88 (dd, J (3,4)=16, J (4,1')=6, h - C (4));

IR: (flüssig) 1700, 1680, 1620, 1360, 1250 cm $^{-1}$

MS: $M^+$ 206 (1), 191 (17), 163 (63), 149 (8), 135 (11), 121 (41), 107 (47), 93 (33), 83 (33), 79 (27), 70 (22), 55 (55), 43 (100).

Geruch des Gemisches der zwei Verbindungen:

nach frischen Himbeeren, iononartig, holzig, Kopfnote fruchtig.

## Beispiel 4

Eine Lösung von 30 g eines Gemisches von 1,5,5,6-Tetramethyl-cyclcohex-1-en und 1,2,6,6-Tetramethyl-cyclohex-1-en, im Verhältnis 1:2, und 14,7 g 3-Butin-2-on in 200 ml Methylenchlorid werden in Gegenwart von 15 g Zinkchlorid drei Tage bei Raumtemperatur gerührt. Dann wird mit Wasser gewaschen, über Natriumsulfat getrocknet und eingedampft. Der Rückstand (45 g) wird destilliert. Man erhält auf diese Weise 21 g eines Gemisches der nachstehenden Verbindungen:

4-(1',6',6'-Trimethyl-2'-methylen-cyclohex-1'-yl)-but-3-en-2-on,
4-(1',2',6',6'-Tetramethyl-cyclohex-2-en-1'-yl)-but-3-en-2-on,
4-(3',4',4'-Trimethyl-2'-methylen-cyclohex-1'-yl)-but-3-en-2-on,
4-(2',3',4',4'-Tetramethyl-cyclohex-2-en-1'-yl)-but-3-en-2-on,
4-(1',3',3'-Trimethyl-2'-methylen-cyclohex-1'-yl)-but-3-en-2-on.

Spektrale Daten des Gemisches:

NMR ($CDCl_3$ 400 MHz): $-CH_3$: 0,695; 0,8; 0,875; 0,897; 0,995 (1); 1,105;

$-COCH_3$: 2,257; 2,26; 2,265; 2,275; 2,295.

## Beispiel 5

Eine Lösung von 44,6 g eines Gemisches von 1,3,3,5-Tetramethyl-cyclohex-1-en und 1,3,5,5-Tetramethyl-cyclohex-1-en und 22 g 3-Butin-2-on in 250 ml Methylenchlorid wird in Gegenwart von 66 g Zinkchlorid 5 Tage gerührt. Das Reaktionsgemisch wird hierauf auf Wasser gegossen, mit Aether ausgeschüttelt und mit einer gesättigten Kochsalzlösung gewaschen. Die organische Phase wird über Magnesiumsulfat getrocknet und eingedampft. Der Rückstand (62,1 g) wird am Hochvakuum destilliert. Man erhält auf diese Weise 39,6 g eines Gemisches von 4-(2',4',6',6'-Tetramethyl-cylcohex-2'-en-1'-yl)-but-3-en-2-on, 4-(4',6',6'-Trimethyl-2'-methylen-cyclohex-1'-yl)-but-3-en-2-on, 4-(2',4',4',6'-Tetramethyl-cyclohex-2'-en-1'-yl)-but-3-en-2-on und 4-(4',4',6'-Trimethyl-2'-methylen-cyclohex-1'-yl)-but-3-en-2-on.

NMR ($CDCl_3$ 400 MHz): $-CH_3$: 0,825; 0,855; 0,865;
des Gemisches 0,875; 0,8925; 0,945; 0,965; 0,985; 0,99.

Geruch des Gemisches: im Fond iononartig, staubig, wurzelig, nelkenartig.

## Beispiel 6

a) Aus 3 g Magnesium und 17,2 g Methyljodid in 35 ml Aether wird eine Grignardlösung hergestellt. Dazu werden 13,1 g 3,4,4-Trimethyl-cyclohexanon, gelöst in 20 ml Aether, während 25 Minuten zugetropft. Das Gemisch wird noch 4 Stunden bei Rückflusstemperatur gehalten und dann

12 Stunden stehengelassen. Hierauf wird das Reaktionsgemisch auf Eis gegossen und mit total 55 ml 2 N HCl auf einen pH-Wert von 2 gestellt. Es wird mit Aether extrahiert, die vereinigten organischen Phasen mit gesättigter Kochsalzlösung und Natriumbicarbonatlösung neutral gewaschen, über Magnesiumsulfat getrocknet und eingeengt. Der Rückstand (13,9 g) wird über 150 g Kieselgel 60 Merck (Korngrösse 0,04 - 0,063 mm) filtriert. Durch Elution mit 30% Aether in Hexan erhält man 8,2 g 1,3,4,4-Tetramethyl-cyclohexan-1-ol.

NMR ($CDCl_3$ 400 MHz): $-CH_3$: 0,73; 0,78; 0,85; 0,9; 1,16; 1,23.

b) 7,6 g 1,3,4,4-Tetramethyl-cyclohexan-1-ol werden in Gegenwart von 2,9 g Oxalsäure 1 Stunde auf 130°C erhitzt. Das Produkt wird anschliessend über eine Vigreuxkolonne destilliert. Man erhält auf diese Weise 5,4 g eines Gemisches von 1,3,4,4-Tetramethyl-cyclohex-1-en und 1,4,4,5-Tetramethyl-cyclohex-1-en.

NMR ($CDCl_3$ 400 MHz): $-CH_3$: 0,725; 0,737; 0,825; 0,845; 0,84; 0,855; 0,885; 0,905;

CH=C: 1,095; 1,26.

c) Ein Gemisch von 4 g 1,3,4,4-Tetramethyl-cyclohex-1-en und 1,4,4,5-Tetramethyl-cyclohex-1-en, gelöst in 40 ml Methylenchlorid wird 10 Tage bei Raumtemperatur in Gegenwart von 2,2g 3-Butin-2-on und 5,9 g Zinkchlorid gerührt, dann auf Wasser gegossen, mit Aether extrahiert, über Magnesiumsulfat getrocknet und eingeengt. Der Rückstand (5,5 g) wird über 200 g Kieselgel 60 Merck (Korngrösse 0,04 - 0,063 mm) chromatographiert. Elution mit 5% Aether in Hexan ergibt 1,35 g eines Isomerengemisches von:

4-(4',5',5'-Trimethyl-2'-methylen-cyclohex-1'-yl)-but-3-en-2-on,

4-(2',4',5',5'-Tetramethyl-cyclohex-2'-en-1'-yl)-but-3-en-2-on,

4-(5',5',6'-Trimethyl-2'-methylen-cyclohex-1'-yl)-but-3-en-2-on,

4-(2',5',5',6'-Tetramethyl-cyclohex-2'-en-1'-yl)-but-3-en-2-on.

NMR ($CDCl_3$ 400 MHz) $-CH_3$: 0,74; 0,76; 0,81; 0,827; 0,877; 0,895; 0,93;

$-CO-CH_3$: 2,25; 2,265; 2,275; 2,28; 2,29.

Geruch: nach Iris, wurzelig.

Mit demselben Lösungsmittel werden nachträglich noch 1,8 g 4-(2',4',5',5'-Tetramethyl-cyclohex-1'-en-1'-yl)--but-3-en-2-on eluiert.

NMR ($CDCl_3$ 400 MHz): 0,747 (3H, s); 0,862 (3H, d J= 0,96, 3H, s); 1,912 (3H, s); 2,290 (3H, s); 6,095 (1H, d J =16); 7,7 (1H, d J = 16).

Geruch: nach Myrrhe, nach Opponax.

## Beispiel 7

a) 32,3 g 1,2,2,5-Tetramethyl-cyclohexan-1-ol, gelöst in 160 ml Benzol werden in Gegenwart von 2 g Toluol-4-sulfonsäure am Wasserabscheider erhitzt. Hierauf wird abgekühlt, mit Wasser gewaschen, getrocknet, eingedampft und

destilliert. Man erhält auf diese Weise 22,5 g eines Gemisches von hauptsächlich 2,3,3,6-Tetramethyl-cyclohex-1-en.

NMR ($CDCl_3$ 400 MHz) $-CH_3$: 0,905; 0,920, 0,922; 0,975; 0,980, 0,992; 1,015; 1,625

$C=CH_2$, $-CH=C$: 5,135; 5,270.

b) Eine Lösung von 12,2 g des obigen, 2,3,3,6-Tetramethyl-cyclohex-1-en enthaltenden Gemisches und 7,32 g 3-Butin-2-on, gelöst in 100 ml Methylenchlorid wird in Gegenwart von 7,32 g Zinkchlorid 5 Tage bei Raumtemperatur gerührt. Hierauf wird das Reaktionsgemisch auf Eis gegossen, mit Wasser gewaschen, über Calciumchlorid getrocknet und eingedampft. Das Rohprodukt wird am Hochvakuum destilliert: Man erhält auf diese Weise 9,3 g eines Produktes, das hauptsächlich aus trans-trans-4-(2'-Methylen-3',3',6'-trimethyl-cyclohex-1'-yl)-but-3-en-2-on besteht.

NMR ($CDCl_3$ 400 MHz): 0,92 (3H, d J=6); 0,995 (3H,s); 1,105 (3H, s); 2,255 (3H, s); 4,82 (1H, s); 4,92 (1H, d J = 2); 6,11 (1H, dd, J=16, J=2); 6,94 (1H, dd, J=5, J=16).

Beispiel 8

a) Aus 19 g Magnesium und 111 g Methyljodid, gelöst in 280 ml Aether wird eine Grignardlösung hergestellt. Dazu werden 100 g 2,4,4-Trimethyl-cyclohexanon, gelöst in 100 ml Aether, während 45 Minuten zugetropft. Das Gemisch wird noch 2 Stunden bei Rückflusstemperatur gehalten und hierauf 12 Stunden bei Raumtemperatur stehengelassen. Es

wird auf Eis gegossen, mit 10%-iger Schwefelsäure neutralisiert und die wässrige Phase viermal mit Aether extrahiert. Die organischen Phasen werden mit gesättigter Natriumbicarbonatlösung und Wasser neutral gewaschen, über Natriumsulfat getrocknet und eingedampft. Man erhält auf diese Weise 115 g rohes 1,2,4,4-Tetramethyl-cyclohexan-1-ol.

NMR ($CDCl_3$ 60 MHz): 0,809; 0,928; 1,04; 1,190.

b) 105 g rohes 1,2,4,4-Tetramethyl-cyclohexan-1-ol werden in Gegenwart von 10 g Oxalsäure unter Normaldruck destilliert. Das Destillat wird mit 10% Bicarbonatlösung gewaschen und noch ein zweites Mal destilliert. Man erhält auf diese Weise 62,6 g eines Reaktionsproduktes, das hauptsächlich aus 1,2,4,4-Tetramethyl-cyclohex-1-en besteht.

NMR ($CDCl_3$ 60 MHz): 0,88 (6H, s); 1,571 (3H, s); 1,62 (3H, s).

c) Eine Lösung von 30 g 1,2,4,4-Tetramethyl-cyclohex-1-en und 18 g 3-Butin-2-on, gelöst in 300 ml Methylenchlorid, wird in Gegenwart von 18 g Zinkchlorid zwei Tage bei Raumtemperatur gerührt. Alsdann wird auf Wasser gegossen, mit einer gesättigten Natriumcarbonatlösung gewaschen, über Natriumsulfat getrocknet und eingedampft. Das Rohprodukt (49g) wird am Hochvakuum destilliert. Man erhält auf diese Weise 27,5 g eines Gemisches von 4-(1',2',4',4'-Tetramethyl-cyclohex-2'-en-1'-yl)-but-3-en-2-on,

NMR ($CDCl_3$ 400 MHz): 0,952 (3H, s); 0,962 (3H, s); 1,175 (3H, s); 1,540, 3H, d, J = 1); 2,260 (3H, s); 5,265 (1H, s);

5,935 (1H, d, J=16); 6,637 (1H, d, J=16).

und von 4-(1',4',4'-Trimethyl-2'-methylen-cyclohex-1'-yl)-but-3-en-2-on.

NMR ($CDCl_3$ 400 MHz): 0,855 (3H, s); 0,890 (3H, s); 1,195 (3H, s); 2,265 (3H, s); 4,785 1H, s); 4,807 (1H, s); 6,037 (1H, d, J=16); 6,805 1H, d, J=16).

Geruch des Gemisches: holzig, fruchtig, camphrig, iononartig, gut haftend.

## Beispiel 9

Es werden die 4 folgenden Parfumbasen erstellt:

a) Chypre 1

| | Gewichtsteile |
|---|---|
| Vanillin | 20 |
| Musk moskene (1,1,3,3,6-Pentamethyl--5,7-dinitro-dihydroinden) | 20 |
| Givescone (2-Aethyl-6,6-dimethyl-2-cyclohexen-1-carbonsäureäthylester) | 20 |
| Phenyläthylalkohol | 20 |
| Sandalore (3-Methyl-5-(2,2,3-trimethyl-cyclopent-3-en-1-yl)pentan-2-ol) | 30 |
| Patchouliöl | 30 |
| Ketonmoschus | 30 |
| Benzylacetat | 50 |
| Hedione (Methyldihydrojasmonat) | 50 |
| Coumarin rein krist. | 80 |
| Eichenmoos absolut yougosl. | 100 |
| Bergamotteöl | 250 |
| α-Hexylzimtaldehyd | 100 |

Es wurden in diese Komposition die Produkte der Beispiele 3c, 4, 5, 6c, 8c und 9c eingearbeitet. In jedem der Fälle wurde der blumige, holzige Aspekt dieser Komposition stark akzentuiert. Am eindrücklichsten war der Effekt mit den Produkten der Beispiele 3c, 4 und 6c.

b) Chypre 2

| | Gewichtsteile |
|---|---|
| Lemonile (3,7-Dimethyl-nonadien(2,6)-säurenitril) | 1 |
| Stemone (3-Methyl-heptan-5-on-oxim) | 2 |
| Aurantiol rein nat. (Methyl-N-3,7-dimethyl-7-hydroxyoctyliden-anthranilat) | 2 |
| Viridine(1,1-Dimethoxy-2-phenylaethan) | 5 |
| Fraise pure (α-Methylphenyläthylglycidat) 10% in Dipropylenglykol | 5 |
| Citronensäure | 5 |
| Rosacetol (Trichlormethylphenylcarbinylacetat) | 5 |
| Resedal (4,4,6-Trimethyl-2-benzyl-1,3-dioxan) | 5 |
| Epoxycedren | 10 |
| Acetyl-isoeugenol | 10 |
| Vanillin | 10 |
| Phenyläthylalkohol | 20 |
| Musk moskene | 20 |
| Givescone | 20 |
| Isoraldein (Isomethyl-α-ionon) | 30 |
| Ketonmoschus | 30 |
| Sandalore | 30 |
| Patchouliöl | 30 |
| l-Limonen | 40 |
| Benzylacetat extra | 50 |
| Hedione | 50 |

| | |
|---|---|
| Coumarin rein krist. | 80 |
| Eichenmoos absolut | 100 |
| α-Hexylzimtaldehyd | 100 |
| Bergamotteöl | 140 |

Durch Zugabe von 200 g des Produktes des Beispiels 6c wurden die holzigen und irisartigen Noten der Komposition verstärkt und bzgl. Kraft, Volumen und Diffusion der Komposition in eindrücklicher Weise gesteigert.

c) Parfum Oriental

| | Gewichtsteile |
|---|---|
| Viridine | 1 |
| Diphenyloxid | 1 |
| Prunolid (γ-Nonalacton) 10 % in Dipropylenglykol | 1 |
| Fraise pure 10% in Dipropylenglykol | 1 |
| Aurantiol rein nat. | 1 |
| Vanillin | 5 |
| Sandalore | 10 |
| Benzylacetat extra | 10 |
| Terpineol | 10 |
| Ambrettolide (Cyclohexadecen-7-olid) | 10 |
| Hydroxycitronellal | 10 |
| Nerol extra | 10 |
| Dimethylbenzylcarbinylacetat | 10 |
| Ketonmoschus | 10 |
| l-Limonen | 10 |
| Epoxy-cedren | 10 |
| Bergamotteöl | 15 |
| Lavendelöl | 15 |
| Lilial | 15 |
| Citronellol extra | 15 |
| α-Hexylzimtaldehyd | 20 |

| | |
|---|---|
| Geraniumöl Bourbon | 20 |
| Ylang-Ylang-Oel extra | 20 |
| Crysolide (6-tert. Butyl-1,1-dimethyl--4-acetylindan) | 20 |
| Jasmonyl (Nonandiol-1,3-acetat) | 50 |
| Sandelholzöl | 70 |
| Eichenmoos absolue | 80 |
| Benzylsalicylat | 200 |

Durch Zugabe von 150 g des Produktes des Beispiels 3c wurde die Kraft der Komposition erhöht und die Vanille-, Holz- und Moschusnoten massiv unterstützt

d) Parfum Floral

| | Gewichtsteile |
|---|---|
| Nootkaton krist. (4α-Methyl-4aα-methyl-6ß-isopropenyl-2,3,4,4a,5,6,7,8-octahydro-2-naphthalenon) | 1 |
| Lemonile | 1 |
| Verdanthiol (Methyl-N-(p.-tert. butyl-α-methyl-hydrozimt)anthranilat) | 1 |
| Fraise pure 10% in Dipropylenglykol | 1 |
| Nonadienal 1% in Dipropylenglykol | 2 |
| Diphenyloxid | 2 |
| Methyloctincarbonat | 5 |
| Vanillin | 5 |
| Acetal E (1-Phenyl-4-methyl-3,5-dioxy-heptan) | 5 |
| Rosacetol | 5 |
| Oxyoctalinformiat (1,2,3,4,4a,7,8,8a-octahydro-2,4a,5,8a-tetramethyl-1-naphthylformiat) | 10 |
| Folenox (Isolongifolenepoxyd) | 10 |
| Ketonmoschus | 20 |

| | |
|---|---|
| Vetiveröl Haiti | 20 |
| Benzylisoeugenol | 20 |
| 1-Limonen | 20 |
| Ylang-Ylang-Oel | 40 |
| Isoraldein | 50 |
| Heliotropin | 50 |
| Benzylacetat extra | 50 |
| α-Hexylzimtaldehyd | 60 |
| Bergamotteöl | 100 |
| Hedione | 100 |
| Stemone | 2 |
| Citronellol extra | 20 |

Durch Zugabe von 400 g des Produktes des Beispiels 4 wurde die Veilchennote der Komposition in eindrücklicher Weise akzentuiert und Fülle und Reichtum der Komposition nahmen beträchtlich zu.

## Patentansprüche

1. Verfahren zur Herstellung von mehrheitlich in der trans-Form vorliegenden Verbindungen der Formel

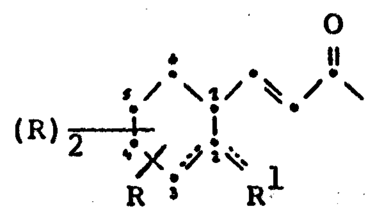

I

worin das Symbol R für eine Methylgruppe steht, $R^1$ für Methyl oder Methylen steht, $(R)_2$ zwei Methylgruppen in geminaler Stellung in 3,4,5 oder 6-Stellung des Moleküls darstellt und eine der gestrichelt gezeichneten Linien eine zusätzliche Bindung ist,

dadurch gekennzeichnet, dass man eine Verbindung der Formel

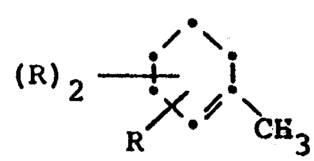

II

worin R und $(R)_2$ obige Bedeutungen besitzen,

in Gegenwart einer schwachen Lewis-Säure mit 3-Butin-2-on behandelt.

2. Verfahren nach Anspruch 1, dadurch gekennzeichnet, dass man Zinkchlorid, Zinkjodid oder Zinkbromid als Lewis-säure verwendet.

3. Mehrheitlich in der trans-Form vorliegende Verbindungen der Formel

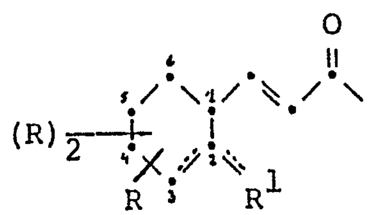

I

worin R, $R^1$, $(R)_2$ und die gestrichelt gezeichneten Linien obige Bedeutungen besitzen,
mit der Ausnahme von α-Iron und γ-Iron.

4. Gemisch von 4-(2',4',6',6'-Tetramethyl-cyclohex-2'-en-1'-yl)-but-3-en-2-on, 4-(4',6',6'-Trimethyl-2'-methylen-cyclohex-1'-yl)-but-3-en-2-on, 4-(2',4',4',6'-Tetramethyl-cyclohex-2'-en-1'-yl)-but-3-en-2-on und 4-(4',4',6'-Trimethyl-2'-methylen-cyclohex-1'-yl)-but-3-en-2-on.

5. Gemisch von 4-(1',2',4',4'-Tetramethyl-cyclohex-2'-en-1'-yl)-but-3-en-2-on und 4-(1',4',4'-Trimethyl-2'-methylen-cyclohex-1'-yl)-but-3-en-2-on.

6. trans-trans-4-(2'-Methylen-3',3',6'-trimethyl-cyclohex-1'-yl)-but-3-en-2-on.

7. Gemisch von 4-(1',6',6'-Trimethyl-2'-methylen-cyclohex-1'-yl)-but-3-en-2-on, 4-(1',2',6',6'-Tetramethyl-cyclohex-2'-en-1'-yl)-but-3-en-2-on, 4-(3',4',4'-Trimethyl-2'-methylen-cyclohex-1'-yl)-but-3-en-2-on, 4-(2',3',4',4'-Tetramethyl-cyclohex-2'-en-1'-yl)-but-3-en-2-on und 4-(1',3',3'-Trimethyl-2'-methylen-cyclohex-1'-yl)-but-3-en-2-on.

8. Gemisch von trans-trans-4-(2',4',4'5'-Tetramethyl-cyclohex-2'-en-1'-yl)-but-3-en-2-on und trans-trans-4-(4',4',5'-Trimethyl-2'-methylen-cyclohex-1'-yl)-but-3-en-2-on.

9. Gemisch von 4-(4',5',5'-Trimethyl-2'-methylen-cyclohexan-1'-yl)-but-3-en-2-on, 4-(2',4',5',5'-Tetramethyl-cyclohex-2'-en-1'-yl)-but-3-en-2-on, 4-(5',5',6'-Trimethyl-2'-methylen-cyclohex-1'-yl)-but-3-en-2-on und 4-(2',5',5',6'-Tetramethyl-cyclohex-2'-en-1'-yl)-but-3-en-2-on.

10. Riechstoffkomposition, gekennzeichnet durch einen Gehalt an einer mehrheitlich in der trans-Form vorliegenden Verbindung der Formel

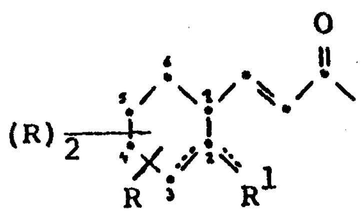

I

worin R, $R^1$, $(R)_2$ und die gestrichelt gezeichneten Linien obige Bedeutung besitzen,

mit der Ausnahme von α-Iron und γ-Iron.

11. Verwendung von mehrheitlich in der trans-Form vorliegenden Verbindungen der Formel

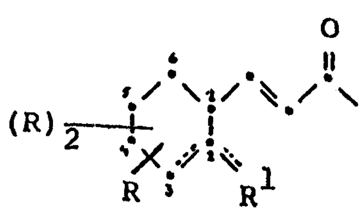

I

worin R, $R^1$, $(R)_2$ und die gestrichelt gezeichneten Linien obige Bedeutung besitzen,

mit der Ausnahme von α-Iron und γ-Iron,

als Riechstoffe.

* * *

Europäisches Patentamt

# EUROPÄISCHER RECHERCHENBERICHT

0210391

Nummer der Anmeldung

EP 86 10 7897

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | FR-A-2 016 610 (BASF)<br>* Anspruch; Seiten 1-3, Zeilen 27-28 *<br>--- | 3,4,9-11 | C 07 C 175/00<br>C 11 B 9/00<br>A 61 K 7/46 |
| Y | GB-A-2 144 118 (INSTITUT KHIMII AKADEMII NAUK ESTONSKOI SSR (USSR))<br>* Anspruch; Seite 1, Zeilen 5-7 *<br>& DE-A-3 327 304<br>----- | 3,10,11 | |
| | | | **RECHERCHIERTE SACHGEBIETE (Int. Cl.4)** |
| | | | C 07 C 175/00 |

Der vorliegende Recherchenbericht wurde für alle Patentansprüche erstellt.

| Recherchenort | Abschlußdatum der Recherche | Prüfer |
|---|---|---|
| DEN HAAG | 29-10-1986 | BONNEVALLE E.I.H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument